# EUROPEAN PATENT SPECIFICATION

(11) **EP 2 603 238 B1**
(45) Date of publication and mention of the grant of the patent: **30.07.2014**
(21) Application number: 11740931.8
(22) Date of filing: 08.08.2011
(51) Int. Cl.: A61K 41/00, A61K 48/00, A61P 27/02, A61K 9/00, C12N 15/87

(54) **METHODS AND PHARMACEUTICAL COMPOSITIONS FOR THE TREATMENT OF AN OCULAR DISEASE IN A SUBJECT**
VERFAHREN UND PHARMAZEUTISCHE ZUSAMMENSETZUNGEN ZUR BEHANDLUNG EINER AUGENERKRANKUNG BEI EINEM PATIENTEN
PROCÉDÉS ET COMPOSITIONS PHARMACEUTIQUES POUR LE TRAITEMENT D'UNE MALADIE OCULAIRE CHEZ UN SUJET

(30) Priority: 09.08.2010 EP 10305873
(43) Date of publication of application: 19.06.2013
(73) Proprietor: INSERM (Institut National de la Santé et de la Recherche Médicale), 75013 Paris (FR)
(72) Inventor: BEHAR-COHEN, Francine, F-75270 PARIS Cedex 06 (FR)
(74) Representative: Nony
(86) International application number: PCT/EP2011/063631
(87) International publication number: WO 2012/020001

(56) References cited:
- WO-A2-2006/123248
- BLOQUEL C ET AL: "Non-viral ocular gene therapy: Potential ocular therapeutic avenues", ADVANCED DRUG DELIVERY REVIEWS, ELSEVIER BV, AMSTERDAM, NL LNKD- DOI:10.1016/J.ADDR.2006.07.023, vol. 58, no. 11, 15 November 2006 (2006-11-15), pages 1224-1242, XP024892110, ISSN: 0169-409X [retrieved on 2006-11-15]
- SONODA S ET AL: "Gene transfer to corneal epithelium and keratocytes mediated by ultrasound with microbubbles", INVESTIGATIVE OPHTHALMOLOGY AND VISUAL SCIENCE 200602 US LNKD- DOI:10.1167/IOVS.05-0889, vol. 47, no. 2, February 2006 (2006-02), pages 558-564, XP002604883, ISSN: 0146-0404

## Description

### FIELD OF THE INVENTION:

The present invention relates to pharmaceutical compositions for the treatment of an ocular disease in a subject.

### BACKGROUND OF THE INVENTION:

In recent years, there have been exciting new advances for the treatment of blinding ocular diseases such as age-related macular degeneration and diabetic retinopathy, using biotherapies.

Repeated intravitreal injections of therapeutic proteins are rapidly increasing in frequency as a route of intraocular delivery of therapeutic proteins. Intravitreous injections are commonly performed and well tolerated, but they are associated with rare but severe complication risks such as retinal lesions, cataract formation and eye infection. More problematic is the poor compliance of frequently repeated injections. A wide field of innovative methods is being explored to reduce the frequency of intravitreous injections.

Gene therapy is an optimal option for eye diseases and particularly non-viral gene vectors are promising to pay no heed limits of viral vectors which are associated with immunogenicity, toxicity and risk of insertion in oncogenic sequences. Moreover, non viral vectors are more appropriate for the control of the doses of the therapeutic proteins which might be a crucial factor for efficacy and specificity of such therapeutic factors. For example; eye tissues are a recently developed target for in vivo gene transfer. In this context, Behar-Cohen et al (2006) have developed a novel in vivo electrotransfer (ET) technique in the rat eye using for the first time the ciliary muscle as a target tissue for gene delivery and as a reservoir for production of therapeutic proteins into the ocular media: aqueous and vitreous humours (WO 2006123248). Its efficacy was demonstrated by evaluating the effects of intraocular production of TNF receptors in two rat models of ocular inflammation (uveitis) (Bloquel C et al. Plasmid electrotransfer of eye ciliary muscle: principles and therapeutic efficacy using hTNF-alpha soluble receptor in uveitis. FASEB J. 2006; 20: 389-391.; Kowalczuk L et al. Local ocular immunomodulation resulting from electrotransfer of plasmid encoding soluble TNF receptors in the ciliary muscle. Invest Ophthalmol Vis Sci 2009; 50: 1761-1768.; ouchard E et al. Effects of ciliary muscle plasmid electrotransfer of TNF-α soluble receptor variants in experimental uveitis. Gene Ther 2009; 16: 862-873.).

Although electrotransfer to the ciliary muscle is an effective physical method of gene transfer, it requires invasive needle electrode placement into the target tissue to deliver electric pulses. Accordingly, there is a need in the art for methods that are less invasive. There has been no previous study of whether low-intensity ultrasound exposure combined with commercial microbubble is able to increase gene transfer into the smooth ciliary muscle after intramuscular delivery of reporter genes.

### SUMMARY OF THE INVENTION:

The present invention relates to a pharmaceutical composition for use in a method for treating an ocular disease in a subject comprising the steps consisting of i) delivering a pharmaceutical composition formulated with echo-contrast agent microbubbles and a therapeutic nucleic acid of interest into the ciliary muscle of the subject and ii) exposing the region where the pharmaceutical composition was delivered to ultrasound to induce transfection of said therapeutic nucleic acid of interest into said ciliary muscle.

### DETAILED DESCRIPTION OF THE INVENTION:

The aim of the inventors was to assess the feasibility of using low-intensity ultrasound (US) combined with commercially available echo-contrast agent microbubbles (MB) to specifically transfect the ciliary muscle, which has been previously demonstrated to have the potential for secretion of therapeutic proteins into the ocular media. Therefore, reporter plasmid DNA encoding Gaussia luciferase and containing the *LacZ* gene alone or mixed with 50 % echo-contrast microbubbles (Artison), were injected into rat eye ciliary muscle, with or without ultrasound exposure (1 MHz, 2W/cm2 Isata for 2 minutes, 50% duty cycle). Seven days after transfection, luciferase activity in ocular media was significantly higher in eyes injected with plasmid and treated with MB and US as compared with eyes treated with US alone or with eyes receiving only the plasmid injection. US plus microbubbles showed a 2.6-fold increase in luminescence (p < 0.023) compared with the control non-US treated eyes. Thirty days after transfection, a significant decrease in luciferase activity was observed in each experimental group. Histochemical staining demonstrated β-galactosidase expression in the ciliary region but not limited to the plasmid injection site. Accordingly the inventors demonstrate for the first time in vivo the feasibility of gene transfer, mediated by ultrasound and microbubbles in the ocular tissues for the intraocular production of secreted proteins in the ocular media. These results indicate microbubbles combined with US can be used efficiently to transfect ciliary muscle cells and therefore for the treatment of ocular diseases.

The invention thus relates to compositions for use in such a method for the treatment of an ocular disease in a subject.

More particularly, the present invention relates to a pharmaceutical composition for use in a method for treating an ocular disease in a subject comprising the steps consisting of i) delivering a pharmaceutical composition formulated with echo-contrast agent microbubbles and a therapeutic nucleic acid of interest into the ciliary muscle of the subject and ii) exposing the region where the pharmaceutical composition was delivered to ultrasound to induce transfection of said therapeutic nucleic acid of interest into said ciliary muscle.

The nucleic acid to be used in the instant invention can be any nucleic acid of interest exhibiting a biological property. More particularly, the nucleic acid can be any nucleic acid encoding a natural, truncated, artificial, chimeric or recombinant product [e.g., a polypeptide of interest (including a protein or a peptide), a RNA, etc.] exhibiting a biological activity.

The nucleic acid is preferably a desoxyribonucleic acid (DNA) molecule (cDNA, gDNA, synthetic DNA, artificial DNA, recombinant DNA, etc.) or a ribonucleic acid (RNA) molecule (mRNA, tRNA, RNAi, RNAsi, catalytic RNA, antisens RNA, viral RNA, etc.). The nucleic acid may be single stranded or multiple stranded nucleic acid, preferably double-stranded nucleic acid or may be complexed. The nucleic acid may comprise hybrid sequences or synthetic or semi-synthetic sequences. It may be obtained by any technique known to persons skilled in the art, and especially by screening libraries, by chemical synthesis, or alternatively by mixed methods including chemical or enzymatic modification of sequences obtained by screening libraries.

In a particular embodiment, the therapeutic nucleic acid is of synthetic or biosynthetic origin, or extracted from a virus or from a unicellular or pericellular eukaryotic or prokaryotic organism.

The therapeutic nucleic acid used in the present invention may be naked, may be complexed to any chemical, biochemical or biological agent, may be inserted in a vector, etc., when administered to the ciliary muscle.

As used herein, the term "naked DNA" refers to any nucleic acid molecule which is not combined to a synthetic, biosynthetic, chemical, biochemical or biological agent improving the delivery or transfer of said DNA, or facilitating its entry into the cell.

As used herein, the term "vector" refers to a nucleic acid molecule capable of transporting another nucleic acid to which it has been linked. This term also refers in the present application to any delivery carrier, such as a composition associated to a therapeutic or prophylactic nucleic acid in order to increase its cellular delivery.

Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer to circular double stranded DNA loops which, in their vector form, are not bound to the chromosome. In the present invention, the plasmid is the most commonly used form of vector. The plasmid is a preferred form of naked DNA according to the invention.

Vectors may also be episomal DNA, yeast artificial chromosomes, minichromosomes or viral vectors wherein the viral vector is selected from the group consisting of a lentivirus, an adenovirus, an adeno-associated virus and a virus-like vector.

The vector may also be a lipid vesicle such as a liposome. Lipid based compounds which are not liposomes may further be used. For example, lipofectins and cytofectins are lipid-based positive ions that bind to negatively charged nucleic acid and form a complex that can ferry the DNA across a cell membrane. The invention is intended to include such other forms of expression vectors which serve equivalent functions and which become known in the art subsequently hereto.

In addition, the nucleic acid according to the invention may also contain one or more additional regions, for example regulatory elements of small or large size which are available to the skilled artisan such as a promoter region (constitutive, regulated, inducible, tissue-specific, etc.), for example sequences allowing and/or promoting expression in the ciliary muscle, a transcription termination signal, secretion sequences, an origin of replication and/or nuclear localization signal (nls) sequences which further enhance polynucleotide transfer to the cell nucleus. Such nls sequences have been described in the prior art including the SV40 large T antigen sequence.

Additionally, the nucleic acid may further comprise selectable markers useful in selecting, measuring, and monitoring nucleic acid transfer results (transfer to which tissues, duration of expression, etc.). The types of expression systems and reporter genes that can be used or adapted for use are well known in the art. For example, genes coding for a luciferase activity, an alkaline phosphatase activity, or a green fluorescent protein activity are commonly used.

The nucleic acid according to the invention may contain any nucleotide sequence of any size. The nucleic acid may thus vary in size from a simple oligonucleotide to a larger molecule such as a nucleotide sequence including exons and/or introns and/or regulatory elements of any sizes (small or large), a gene of any size, for example of large size, or a chromosome for instance, and may be a plasmid, an episome, a viral genome, a phage, a yeast artificial chromosome, a minichromosome, an antisense molecule, etc.

In a particularly preferred embodiment, the polynucleotide is a double-stranded, circular DNA, such as a plasmid, encoding a product with biological activity.

The nucleic acid can be prepared and produced according to conventional recombinant DNA techniques, such as amplification, culture in prokaryotic or eukaryotic host cells, purification, etc. The techniques of recombinant DNA technology are known to those of ordinary skill in the art.

In a particular embodiment, the nucleic acid of interest is capable of exerting a beneficial effect on ocular cells. It may compensate for a deficiency in or reduce an excess of an endogenous substance. Alternatively, it may confer new properties on the cells. It may be for example an antisense sequence or nucleic acid encoding a polypeptide which can affect the function, morphology, activity and/or metabolism of ocular cells.

The down regulation of gene expression using antisense nucleic acids can be achieved at the translational or transcriptional level. Antisense nucleic acids of the invention are preferably nucleic acid fragments capable of specifically hybridizing with a nucleic acid encoding an endogenous ocular active substance or the corresponding messenger RNA. These antisense nucleic acids can be synthetic oligonucleotides, optionally modified to improve their stability and selectivity. They can also be DNA sequences whose expression in the cell produces RNA complementary to all or part of the mRNA encoding an endogenous ocular active substance. Antisense nucleic acids can be prepared by expression of all or part of a nucleic acid encoding an endogenous ocular active substance, in the opposite orientation. Any length of antisense sequence is suitable for practice of the invention so long as it is capable of down-regulating or blocking expression of the endogenous ocular active substance. Preferably, the antisense sequence is at least 20 nucleotides in length. The preparation and use of antisense nucleic acids, DNA encoding antisense RNAs and the use of oligo and genetic antisense is disclosed in WO92/15680.

Among the biologically active polypeptides or proteins optionally expressed by a nucleic acid as described above or usable as a biologically active agent and suitable for practice of the invention are enzymes, blood derivatives, hormones, lymphokines, cytokines, chimiokines, anti-inflammatory factors, growth factors, trophic factors, neurotrophic factors, haematopoietic factors, angiogenic factors, anti-angiogenic factors, inhibitors of metalloproteinase, regulators of apoptosis, coagulation factors, receptors thereof, in particular soluble receptors, a peptide which is an agonist or antagonist of a receptor or of an adhesion protein, antigens, antibodies, fragments or derivatives thereof and other essential constituents of the cell.

Various retina-derived neurotrophic factors have the potential to rescue degenerating photoreceptor cells, and may be delivered trough a method according to the present invention. Preferred biologically active agents may be selected from VEGF, Angiogenin, Angiopoietin-1 , DeM, acidic or basic Fibroblast Growth Factors (aFGF and bFGF), FGF-2, Follistatin, Granulocyte Colony-Stimulating factor (G-CSF), Hepatocyte Growth Factor (HGF), Scatter Factor (SF), Leptin, Midkine, Placental Growth Factor (PGF), Platelet-Derived Endothelial Cell Growth Factor (PD- ECGF), Platelet-Derived Growth Factor-BB (PDGF-BB), Pleiotrophin (PTN), RdCVF (Rod-derived Cone Viability Factor), Progranulin, Proliferin, Transforming Growth Factor-alpha (TGF-alpha), Transforming Growth Factor-beta (TGF-beta), Tumor Necrosis Factor-alpha (TNF-alpha), Vascular Endothelial Growth Factor (VEGF), Vascular Permeability Factor (VPF), CNTF, BDNF, GDNF, PEDF, NT3, BFGF, angiopoietin, ephrin, EPO, NGF, IGF, GMF, aFGF, NT5, Gax, a growth hormone, [alpha]-1-antitrypsin, calcitonin, leptin, an apolipoprotein, an enzyme for the biosynthesis of vitamins, hormones or neuromediators, chemokines, cytokines such as IL-1, IL-8, IL-10, IL-12, IL-13, a receptor thereof, an antibody blocking anyone of said receptors, TIMP such as TIMP-1, TIMP-2, TIMP-3, TIMP-4, angioarrestin, endostatin such as endostatin XVIII and endostatin XV, ATF, angiostatin, a fusion protein of endostatin and angiostatin, the C- terminal hemopexin domain of matrix metalloproteinase-2, the kringle 5 domain of human plasminogen, a fusion protein of endostatin and the kringle 5 domain of human plasminogen, the placental ribonuclease inhibitor, the plasminogen activator inhibitor, the Platelet Factor-4 (PF4), a prolactin fragment, the Proliferin-Related Protein (PRP), the antiangiogenic antithrombin III, the Cartilage-Derived Inhibitor (CDI), a CD59 complement fragment, vasculostatin, vasostatin (calreticulin fragment), thrombospondin, fibronectin, in particular fibronectin fragment gro-beta, an heparinase, human chorionic gonadotropin (hCG), interferon alpha/beta/gamma, interferon inducible protein (IP-10), the monokine-induced by interferon-gamma (Mig), the interferon-alpha inducible protein 10 (IP10), a fusion protein of Mig and IP10, soluble Fms-Like Tyrosine kinase 1 (FLT-1) receptor, Kinase insert Domain Receptor (KDR), regulators of apoptosis such as Bcl-2, Bad, Bak, Bax, Bik, BcI-X short isoform and Gax, fragments or derivatives thereof and the like.

In a particular embodiment, the nucleic acid encodes a soluble fragment of the TNF[alpha] receptor, the TGF[beta]2 receptor, of VEGFR-1, VEGFR-2, VEGFR-3, CCR2 or MIP1. The nucleic acid may also, in another preferred embodiment, encode an antibody, a variable fragment of a single-chain antibody (ScFv) or any other antibody fragment having recognition capacities for the purposes of immunotherapy.

In a particular embodiment of the present invention, the biologically active nucleic acid encodes a precursor of a therapeutic protein usable in the present invention such as those described above.

Furthermore, in another embodiment of the present invention, a mixture of nucleic acids encoding distinct biologically active products can be used. This variant allows co-expression of different products in the ocular cells.

The pharmaceutical composition of the invention comprises echo-contrast agent microbubbles composed of a shell filed with a gas core. Typically, said microbubbles have a diameter of 0.1 to 10 microns, but this feature shall not be considered as limiting, and therefore microbubbles having a diameter greater than 10microns or lesser than 0,1 micron are also encompassed by the invention.

An "echo-contrast agent microbubble" as used herein refers to a microsphere comprising a shell with an approximately spherical shape surrounding an internal void comprising a gas. The microbubble "shell" referred to herein refers to a membrane surrounding the internal void of the microbubble and the term "gas" as used herein denotes a substance of low toxicity which either is in the gasous phase at room temperature and normal atmospheric pressure or which can undergo a phase change to the gasous phase at a transition temperature of about 70° C. or lower.

The composition and methods for forming microbubbles as ultrasound contrast agents are well established in the art. Therefore, the person skilled in the art knows the materials and methods to form the microbubbles used in the present invention. Examples of procedures for the preparation of microbubbles are described in: U.S. Pat. No. 4,446,442, U.S. Pat. No. 4,684,479, U.S. Pat. No. 4,718,433, U.S. Pat. No. 5,088,499, U.S. Pat. No. 5,123,414, U.S. Pat. No. 5,271,928, U.S. Pat. No. 5,413,774, U.S. Pat. No. 5,445,813, U.S. Pat. No. 5,556,610, U.S. Pat. No. 5,597,549, U.S. Pat. No. 5,686,060, U.S. Pat. No. 5,773,527, U.S. Pat. No. 5,798,091, U.S. Pat. No. 5,827,504, U.S. Pat. No. 6,217,850, U.S. Pat. No. 6,416,740, U.S. Pat. No. 6,443,898, and European Patent 0458745.

The microbubble shell is a membrane surrounding the internal void of the microbubble. Microbubble shells typically range from about 10 to about 200 nm in thickness and help to prevent destruction and diffusion of the gas core. The microbubble shell typically comprises a surfactant or a polymer. Surfactants suitable for use in microbubble preparation include any compound or composition that aids in the formation and maintenance of a microbubble by forming a layer at the interface between the gas and the medium, usually an aqueous medium, containing the microbubble. The surfactant may comprise a single compound or a combination of compounds. It will be appreciated by the person skilled in the art that a wide range of compounds capable of facilitating formation of the microbubbles can be used in the present invention. The optimum surfactant can be determined through empirical studies that do not require undue experimentation. One practicing the art of the present invention will choose a suitable surfactant based upon such properties as biocompatibility. Preferred surfactants include lipids, including phospholipids and fluorinated lipids. Lipids that may be used include fatty acids; lysolipids; phosphatidylcholines; including dioleoylphosphatidylcholine; dimyristoylphosphatidylcholine, dipentadecanoylphosphatidylcholine, dilauroylphosphatidylcholine, dioleoylphosphatidylcholine, dipalmitoylphosphatidylcholine, and distearoylphosphatidylcholine; phosphatidylethanol amines, including dioleoylphosphatidylethanolamine; phosphatidylserines; phosphatidylglycerol; phosphatidylinositols; sphingolipids; sphingomyelin; glycolipids; ganglioside GM1; ganglioside GM2; glucolipids; sulfatides; glycosphingolipids; phosphatidic acid; palmitic acid; stearic acid; arachidonic acid; oleic acid; lipids bearing polymers such as polyethyleneglycol, chitin, hyaluronic acid or polyvinylpyrrolidone; lipids bearing sulfonated mono-, di-, oligo- or polysaccharides; cholesterol, cholesterol sulfate; cholesterol hemisuccinate; tocopherol hemisuccinate, lipids with ether and ester-linked fatty acids, polymerized lipids, diacetyl phosphate, stearylamine, cardiolipin, phospholipids with short chain fatty acids of 6-8 carbons in length, synthetic phospholipids with asymmetric acyl chains, 6-(5-cholesten-3p-yloxy)-1-thio-(3-D-galactopyranoside, digalactosyldiglyceride, 6-(5-cholesten-3(3yloxy)hexyl-6-amino-6-deoxy-1-thio-(3-D-galactopyranoside, 6-(5-cholesten-3(3-yloxy)hexyl-6-amino-6-deoxyl-lthio-a-D-mannopyranoside, 12-(((7'diethylaminocoumarin-3-yl)carbonyl)methylamino) octadecanoic acid; N-[12-(((7'-diethylaminocoumarin-3-yl) carbonyl)methylamino)octadecanoyl] -2-aminopalmiic acid; cholesteryl (4'-trimethylammonio)butanoate; N-succinyldioleoylphosphatidylethanolamine; 1,2-dioleoyl-sn-glycerol; 1,2-dipalmitoyl-sn-3-succinylglycerol; 1,3-dipalmitoyl-2-succinylglycerol; 1 -hexadecyl-2-palmitoylglycerophosphoethanolamine; palmitoylhomocysteine; and combinations thereof; lauryltrimethylammonium bromide, cetyltrimethylammonium bromide, myristyltrimethylammonium bromide, alkyldimethylbenzylammonium chloride, benzyldimethyldodecylammonium bromide, benzyldimethylhexadecylammonium bromide, benzyldimethyltetradecylammonium bromide, cetyldimethylethylammonium bromide, cetylpyridinium bromide; pentafluoro octadecyl iodide, perfluorooctylbromide, perfluorodecalin, perfluorododecalin, perfluorooctyliodide, perfluorotripropylamine, and perfluorotributylamine. Polymers useful in for use in the present invention include proteins, particularly albumin, particularly human serum albumin, and other biocompatible polymers, including polycyanoacrylate and poly(t-butyloxycarbonylmethyl)glutamate. The nature of the microbubble shell determines the flexibility of the microbubble, the effect of ultrasound, and the binding properties to specific cell types.

Additional components may optionally be included in the microbubble shells to impart desired characterstics to microbubbles. The microbubble may comprise a ligand or targeting molecule included in or bound to the microbubble membrane, wherein the ligand or targeting molecule binds to a target surface or tissue. See, e.g. U.S. Pat. No. 6,245,318; U.S. Pat. No. 6,264,917; P. A. Dayton, et al., "Targeted imaging using ultrasound," J. of Magn. Reson. Imaging, 2002, 16(4), 362-77; S. H. Bloch, et al., "Targeted imaging using ultrasound contrast agents," IEEE Engineering in Medicine and Biology Magazine, 2004, 23(5), 18-29; A. Klibanov, et al, U.S. Pat. Appl. Pub. No. 2005/0260189. Microbubbles incorporating such targeting substances within their shell are included within the scope of the invention. Examples of such targeting molecules that may be included includes: antibodies and antibody fragments, cell adhesion molecules, their receptors, cytokines, growth factors, peptide hormones, peptide mimetics, and fragments thereof; non-peptide agonists/antagonists or non-bioactive binders of receptors for cell adhesion molecules, cytokines, growth factors and peptide hormones; oligonucleotides and modified oligonucleotides; protease substrates or inhibitors; small molecule ligands of biological receptors; inactivated proteases. Where the ligands or targeting molecules are biopolymers, and the methods and compositions of the invention are used in humans, the molecules are preferably human in origin to reduce the possibility of immunogenicity.

Representative classes of gases for inclusion in the microbubble thus include common gases such as air; nitrogen; oxygen; carbon dioxide; hydrogen; inert gases such as helium, argon, xenon or krypton; sulphur fluorides, such as sulphur hexafluoride, disulphur decafluoride or trifluoromethylsulphur pentafluoride; selenium hexafluoride; optionally halogenated silanes such as methylsilane or dimethylsilane; low molecular weight hydrocarbons (e.g. containing up to 7 carbon atoms), for example alkanes such as methane, ethane, a propane, a butane or a pentane, cycloalkanes such as cyclopropane, cyclobutane or cyclopentane, alkenes such as ethylene, propene, propadiene or a butene, and alkynes such as acetylene or propyne; ethers such as dimethyl ether; ketones; esters; halogenated low molecular weight hydrocarbons (e.g. containing up to 7 carbon atoms); and mixtures thereof. Advantageously at least some of the halogen atoms in halogenated gases are fluorine atoms; as in, for example, bromochlorodifluoromethane, chlorodifluoromethane, dichlorodifluoromethane, bromotrifluoromethane, chlorotrifluoromethane, chloropentafluoroethane, dichlorotetrafluoroethane, chlorotrifluoroethylene, fluoroethylene, ethyl fluoride, 1,1-difluoroethane and perfluorocarbons. Other halogenated gases include methyl chloride, fluorinated (e.g. perfluorinated) ketones such as perfluoroacetone and fluorinated (e.g. perfluorinated) ethers, such as perfluorodiethyl ether, and thioethers, such as trifluoromethyl sulfide. Specific examples of gases that may be used in the present invention include: air, allene, argon, bromochlorofluoromethane, bromochlorodifluoromethane, bromodifluoromethane, bromofluoromethane, 3-bromo-1-pentene, bromotrifluoromethane, 1,2-butadiene, 1,3-butadiene, 1-butene, 2-butene, 1-butyne, 2-butyne, carbon dioxide, carbonyl sulfide, 1-chloro-1,1-difluoroethane, 2-chloro-1,1-difluoroethane, 1-chloro-1,1,2,2-tetrafluoroethane, chlorocyclopentene, chlorodifluoromethane, chlorodifluoronitromethane, chloroethane, chlorofluoromethane, 2-chloro-1,1,1,4,4,4hexafluorobutyne, 1 -chloro-1,1,2,2,2-pentafluoroethane, 1 -chloro-1,2,2,2-tetrafluoroethane, chlorotrifluoromethane, cyclobutane, cyclopropane, 1,1,1,2,2,3,4,5,5,5-decafluoropentane, dibromodifluoromethane, 1,2-dibromo-1,1,2,2-tetrafluoromethane, 1,1-dichlorodiacetylene, 1,1-dichloro-2,2difluoroethylene, 1,2-dichloro-1 ,2-difluoroethylene, dichlorodifluoromethane, 1,1-dichloroethane, 1,1-dichloroethylene, dichlorofluoromethane, 1,1-dichloro-1-fluoroethane, 1,1-dichloro-1,2,2,2-tetrafluoroethane, 2,2-dichloro-1, 1,1-trifluoroethane, 1,1-difluoroethane, 1,2-difluoroethane, 1,2-difluoroethylene, difluoromethane, 2,2-difluoropropane, 1,1-dimethylcyclopropane, 1,2-dimethylcyclopropane, dimethylethylamine, 2,3-dimethyl-2-norbornane, 2,2-dimethylpropane, ethane, ethyl cyclopropane, 3-ethyl-3-methyldiaziridine, ethyl vinyl ether, fluoroethane, 1-fluorobutane, helium, 1,1,1,2,3,3,3-heptafluoropropane, hexafluoro-1,3butadiene, 1,1,1,2,3,3-hexafluoro-2,3-dichloropropane, 1,1, 1,3,3,3-hexafluoropropane, hexafluoro-2-methyl-1 -butene, 1,1,1,2,2,3-hexafluoropropane, hexafluoropropylene, iodotrifluoromethane, krypton, 3-methyl-1-butene, 2-methyl-lbutene-3-yne, 3-methyl-1-butyne, methylcyclobutane, methylcyclopropane, isopropylacetylene, methane, 2-methyl-1,3-butadiene, 2-methyl-butane, methyl ether, methyl isopropyl ether, methyl lactate, methyl nitrite, methyl sulfide, methyl vinyl ether, neon, nitrogen, nitrous oxide, 1-nonene-3-yne, octafluoro-2-butene, octafluorocyclobutane, octafluorocyclopentene, oxygen, 1,4-pentadiene, n-pentane, 1,1,1,3,3-pentafluorobutane, pentafluoroethane, 1,1,1,3,3-pentafluoropropane, 1-pentene, E-2-pentene, Z-2pentene, perfluorobutane, perfluoro-1-butene, perfluoro-2butene, perfluoro-2-butyne, perfluorocyclobutene, perfluorodimethylamine, perfluoroethane, perfluoromethane, perfluorohexane, perfluoropentane, perfluoro-1-pentene, perfluoropropane, perfluoropropylene, propane, propene, propyne, selenium hexafluoride, sulfur hexafluoride, tetrafluoroallene, 1,1,1,2-tetrafluoroethane, 1,1,2,2-tetrafluoroethane, tetrafluoromethane, trichlorofluoromethane, trifluoromethylsulfur pentafluoride, 1,1,2-trichloro-1,2,2trifluoroethane, 1,1,1-trifluoroethane, trifluoromethane, vinyl acetylene, vinyl ether, and xenon, and mixtures thereof.

Preferred gases of the invention are fluorocarbon compounds, including: perfluorocarbons, including perfluoroalkanes; hydrofluorocarbons, including hydrofluoroalkanes; chlorofluorocarbons, including chlorofluoroalkanes; hydrochlorofluorocarbons, including hydrochlorofluoroalkanes; and halons (haloalkanes containing bromine, fluorine, and optionally chlorine). Perfluorocarbons, particularly perfluoroalkanes, are particularly preferred. Representative perfluorocarbons include perfluoroalkanes such as perfluoromethane, perfluoroethane, perfluoropropanes, perfluorobutanes (e.g. perfluoro-n-butane, optionally in admixture with other isomers such as perfluoro-iso-butane), perfluoropentanes, perfluorohexanes or perfluoroheptanes; perfluoroalkenes such as perfluoropropene, perfluorobutenes (e.g. perfluorobut-2-ene), perfluorobutadiene, perfluoropentenes (e.g. perfluoropent-1-ene) or perfluoro-4-methylpent-2-ene; perfluoroalkynes such as perfluorobut-2-yne; and perfluorocycloalkanes such as perfluorocyclobutane, perfluoromethylcyclobutane, perfluorodimethylcyclobutanes, perfluorotrimethylcyclobutanes, perfluorocyclopentane, perfluoromethylcyclopentane, perfluorodimethylcyclopentanes, perfluorocyclohexane, perfluoromethylcyclohexane or perfluorocycloheptane. Also preferred is sulfur hexafluoride. Of these gases, perfluoropropane and perfluorobutane are especially preferred because of their demonstrated safety for intraocular injection in humans. They have been used in human studies for intraocular injections to stabilize retinal detachments. Other inert gases such as sulfur hexafluoride are also useful in the invention.

The gas preferably fills at least 50% of the void within the microbubble shell, in some embodiments filling at least 60%, at least 70%, at least 80%, or at least 90% of the void. In some embodiments, the gas substantially fills the void within the microbubble shell.

In addition to the surfactant, it may be desirable to incorporate other agents within the aqueous phase for the formation of microbubbles, or for formulating microbubbles for in vivo use. Such agents may advantageously include conventional viscosity modifiers, buffers such as phosphate buffers or other conventional biocompatible buffers or pH adjusting agents such as acids or bases, and osmotic agents (to provide isotonicity, hyperosmolarity, or hyposmolarity). Preferred solutions have a pH of about 7 and are isotonic. Examples of such agents include sodium chloride and sucrose. Microbubble solutions may be stabilized, for example, by the addition of a wide variety of viscosity modifiers, including, but not limited to carbohydrates and their phosphorylated and sulfonated derivatives; polyethers, preferably with molecular weight ranges between 400 and 8000; di- and trihydroxy alkanes and their polymers, preferably with molecular weight ranges between 800 and 8000. Glycerol, propylene glycol, polyethylene glycol, polyvinyl pyrrolidone, and polyvinyl alcohol may also be useful as stabilizers in the present invention.

Optionally, the microbubbles of the invention may further encapsulate one or more substances. Examples include therapeutic molecules or contrast agents.

Contrast agents include paramagnetic gases, such as atmospheric air, which contains traces of oxygen 17, or paramagnetic ions such as Mn2+, Gd2+, and Fe3+, as well as superparamagnetic particles (ferrites, iron oxides Fe3O4) and may thus be used as susceptibility contrast agents for magnetic resonance imaging (MRI), radioopaque metal ions, such as iodine, barium, bromine, or tungsten, for use as x-ray contrast agents, and gases from quadrupolar nuclei, which may have potential for use as magnetic resonance contrast agents.

Exemplary therapeutic molecules include antineoplastic agents, such as platinum compounds (e.g., spiroplatin, cisplatin, and carboplatin), methotrexate, fluorouracil, adriamycin, mitomycin, ansamitocin, bleomycin, cytosine arabinoside, arabinosyl adenine, mercaptopolylysine, vincristine, busulfan, chlorambucil, melphalan (e.g., PAM, L-PAM or phenylalanine mustard), mercaptopurine, mitotane, procarbazine hydrochloride dactinomycin (actinomycin D), daunorubicin hydrochloride, doxorubicin hydrochloride, taxol, mitomycin, plicamycin (mithramycin), aminoglutethimide, estramustine phosphate sodium, flutamide, leuprolide acetate, megestrol acetate, tamoxifen citrate, testolactone, trilostane, amsacrine (m-AMSA), asparaginase (L-asparaginase) Erwina asparaginase, etoposide (VP-16), interferon a-2a, interferon a-2b, teniposide (VM-26), vinblastine sulfate (VLB), vincristine sulfate, bleomycin, bleomycin sulfate, methotrexate, adriamycin, arabinosyl, hydroxyurea, procarbazine, and dacarbazine; mitotic inhibitors such as etoposide and the vinca alkaloids; radiopharmaceuticals such as radioactive iodine and phosphorus products; hormones such as progestins, estrogens, antiestrogens, growth hormone, melanocyte stimulating hormone, estradiol, beclomethasone dipropionate, betamethasone, betamethasone acetate and betamethasone sodium phosphate, vetamethasone disodium phosphate, vetamethasone sodium phosphate, cortisone acetate, dexamethasone, dexamethasone acetate, dexamethasone sodium phosphate, flunisolide, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, paramethasone acetate, prednisolone, prednisolone acetate, prednisolone sodium phosphate, prednisolone tebutate, prednisone, triamcinolone, triamcinolone acetonide, triamcinolone diacetate, triamcinolone hexacetonide, fludrocortisone acetate, oxytocin, vassopressin, and their derivatives; anti-helmintics, vitamins such as cyanocobalamin retinoic acid, retinoids and derivatives such as retinol palmitate, and a-tocopherol; peptides including enzymes such as manganese super oxide dismutase and alkaline phosphatase; anti-allergic agents such as amelexanox; anti-coagulation agents such as phenprocoumon and heparin; circulatory drugs such as propranolol; metabolic potentiators such as glutathione; antituberculars such as para-aminosalicylic acid, isoniazid, capreomycin sulfate cycloserine, ethambutol hydrochloride ethionamide, pyrazinamide, rifampin, and streptomycin sulfate; antivirals such as acyclovir, amantadine azidothymidine (AZT, DDI, Foscarnet, or Zidovudine), ribavirin and vidarabine monohydrate (adenine arabinoside, ara-A); antianginals such as diltiazem, nifedipine, verapamil, erythritol tetranitrate, isosorbide dinitrate, nitroglycerin (glyceryl trinitrate) and pentaerythritol tetranitrate; antibiotics such as dapsone, chloramphenicol, neomycin, cefaclor, cefadroxil, cephalexin, cephradine erythromycin, clindamycin, lincomycin, amoxicillin, ampicillin, bacampicillin, carbenicillin, dicloxacillin, cyclacillin, picloxacillin, hetacillin, methicillin, nafcillin, oxacillin, penicillin including penicillin G and penicillin V, ticarcillin rifampin and tetracycline; antiinflammatories such as diflunisal, ibuprofen, indomethacin, meclofenamate, mefenamic acid, naproxen, oxyphenbutazone, phenylbutazone, piroxicam, sulindac, tolmetin, aspirin and salicylates; antiprotozoans such as chloroquine, hydroxychloroquine, metronidazole, quinine and meglumine antimonate; antirheumatics such as penicillamine; narcotics such as paregoric; opiates such as codeine, heroin, methadone, morphine and opium; cardiac glycosides such as deslanoside, digitoxin, digoxin, digitalin and digitalis; neuromuscular blockers such as atracurium mesylate, gallamine triethiodide, hexafluorenium bromide, metocurine iodide, pancuronium bromide, succinylcholine chloride (suxamethonium chloride), tubocurarine chloride and vecuronium bromide; sedatives (hypnotics) such as amobarbital, amobarbital sodium, aprobarbital, butabarbital sodium, chloral hydrate, ethchlorvynol, ethinamate, flurazepam hydrochloride, glutethimide, methotrimeprazine hydrochloride, methyprylon, midazolam hydrochloride, paraldehyde, pentobarbital, pentobarbital sodium, phenobarbital sodium, secobarbital sodium, talbutal, temazepam and triazolam; local anesthetics such as bupivacaine hydrochloride, chloroprocaine hydrochloride, etidocaine hydrochloride, lidocaine hydrochloride, mepivacaine hydrochloride, procaine hydrochloride and tetracaine hydrochloride; general anesthetics such as droperidol, etomidate, fentanyl citrate with droperidol, ketamine hydrochloride, methohexital sodium and thiopental sodium; radioactive particles or ions such as strontium, iodide rhenium and yttrium; and prodrugs, such as those disclosed in U.S. Pat. No. 6,443,898.

Typically, an amount of about 10% to about 60% microbubbles are added to the solution containing the nucleic acid of interest.

The pharmaceutical composition of the invention may also comprise compatible or physiologically acceptable carrier, excipient or diluent.

The term "Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

Pharmaceutically compatible or physiologically acceptable carrier, excipient or diluent includes diluents and fillers which are pharmaceutically acceptable for the methods of the invention, are sterile, and may be selected from neutral to slightly acidic, isotonic, buffered saline (including phosphates, chloride, etc.), aqueous or oleaginous solutions or suspensions and more preferably from sucrose, trehalose, surfactants, proteins and amino acids. The pharmaceutically compatible or physiologically acceptable carrier, excipient or diluent is preferably formulated using suitable dispersing, wetting, suspending, soothing, isotonic or viscosity building agents, stabilizers, preservatives and appropriate buffer to form an isotonic solution. The particular pharmaceutically acceptable carrier and the ratio of active compound to carrier are determined by the solubility and chemical properties of the composition, the particular mode of administration, and standard pharmaceutical practice. Those skilled in the art will understand how to formulate such vehicles by known techniques.

An example of stabilizers is disodium edetate or the like. Examples of isotonic agents are glycerin, propylene glycol, polyethylene glycol, sodium chloride, potassium chloride, sorbitol and mannitol or the like. Examples of buffers are citric acid, sodium hydrogenphosphate, glacial acetic acid and trometamol or the like. Examples of pH adjusters are hydrochloric acid, citric acid, phosphoric acid, acetic acid, sodium hydroxide, sodium carbonate and sodium hydrogencarbonate or the like. An example of soothing agents is benzyl alcohol or the like. Examples of preservatives are benzalkonium chloride, benzethonium chloride, p-hydroxybenzoate esters, sodium benzoate and chlorobutanol or the like.

Viscosity greater than that of simple aqueous solutions may be desirable to increase ocular absorption of the active compound, to decrease variability in dispensing the formulations, to decrease physical separation of components of a suspension or emulsion of formulation and/or otherwise to improve the ophthalmic formulation. Such viscosity building agents include, for example, polyvinyl alcohol, polyvinyl pyrrolidone, methyl cellulose, hydroxypropyl methylcellulose, hydroxyethyl cellulose, carboxymethyl cellulose, hydroxypropyl cellulose or other agents known to those skilled in the art. Such agents are typically employed at a level of from about 0.01 to about 2 wt. %.

Preparation forms of the pharmaceutical composition intended for administration to ciliary muscle or cells of subject are preferably liquid preparations. The liquid preparations can be prepared, for example, by dissolving the biologically active agent in BSS (Balanced Salt Solution), a glycerin solution, a hyaluronic acid solution and the like. A particular composition comprises for example BBS (60%) and hyaluronic acid (40%). A stabilizer, an isotonic agent, a buffer, a pH adjustor, a soothing agent, a preservative, electrolytes, such as sodium, potassium, calcium, magnesium and/or chloride or the like can optionally be added in an adequate amount to the liquid preparations.

The pharmaceutical composition may comprise or the biologically active agent may be combined (in a use according to the present invention) with any additional active ingredient or adjuvant. The adjuvant may be selected from any substance, mixture, solute or composition facilitating or increasing the biological activity of the prophylactic or therapeutic agent such as any biologic, synthetic or biosynthetic agent which improves the delivery or transfer of said agent and may be assimilated to a vector (as delivery carrier) according to the invention. The adjuvant may be conditioned and administered separately or sequentially from the prophylactic or therapeutic agent containing composition and/or at a distinct site of injection. Treatment with multiple agents and/or adjuvants according to the invention need not be done using a mixture of agents and/or adjuvants but may be done using separate pharmaceutical preparations. The preparations need not be delivered at the same exact time, but may be coordinated to be delivered to a patient during the same period of treatment, i. e., within a week or a month or each other.

Any suitable therapeutic agents can be coordinated with the compositions of the present invention. Examples of therapeutic agents which may be administered in addition to the above biologically active (prophylactic or therapeutic) agent(s) through a method according to the present invention also include permeabilizing agents such as a virus, a lipid vesicle, hyaluronic acid, lipid-based positive ions, polycationic emulsions, cationic peptides, polyplex, etc.; antibiotics and antimicrobial agents such as tetracycline hydrochloride, leucomycin, penicillin, penicillin derivatives, erythromycin, sulphathiazole and nitrofurazone; local anesthetics such as benzocaine; vasoconstrictors such as phenylephrine hydrochloride, tetrahydrozoline hydrochloride, naphazoline nitrate, oxymetazoline hydrochloride and tramazoline, hydrochloride; cardiotonics such as digitalis and digoxin; vasodilators such as nitro-glycerine and papaverine hydrochloride; antiseptics such as chlorhexidine hydrochloride, hexylresorcinol, dequaliniumchloride and ethacridine; enzymes such as lysozyme chloride and dextranase; hypotensives; sedatives; anti-tumor agents; steroidal anti- inflammatory agents such as hydro-cortisone, prednisone, fluticasone, prednisolone, triamcinolone, acetonide, dexamethasone, betamethasone, beclomethasone, and beclomethasone dipropionate; non-steroidal antiinflammatory agents such as acetaminophen, aspirin, aminopyrine, phenylbutazone, mefanamic acid, ibuprofen, diclofenac sodium, indomethacin, colchicine, and probenocid; enzymatic anti-inflammatory agents such as chymotrypsin and bromelain seratiopeptidase; anti-histaminic agents such as diphenhydramine hydrochloride, chloropheniramine maleate and clemastine; anti-allergic agents; and analgesic compounds.

Actual dosage levels of active ingredients in the compositions of the present invention may be adapted so as to obtain an amount of active ingredient that is effective to obtain a desired biological activity. It should be understood, however, that the specific dose level for any particular patient will depend upon a variety of factors including the body weight, general health, sex, diet, time, rates of absorption and excretion, combination with other drugs and the severity of the particular disease being treated. The pharmaceutical composition of the invention may be delivered into the ciliary muscle at multiple injection sites. The delivering may also be repeated over time.

Ultrasound is high frequency sound, with a frequency of about 10 kHz or greater. Frequencies that are preferred for use in the present invention are those above the range of human hearing, in the frequency range from about 800kHz to about 3MHz. The frequency and intensity of ultrasound used is determined by the requirement to achieve selective microbubble destruction at the site of delivery. The requisite parameters for optimizing microbubble destruction have been studied, and are known to the person skilled in the art. In general, it is expected that ultrasound intensities in the range from about 700 kPa and 200 kPa peak negative pressure will be effective to destroy the microbubbles selectively at the site of delivery. The ultrasound may be applied with any ultrasound device well known in the art. For example, the device may be composed of a focused or unfocused probe that is applied to the ocular surface after interposition of gel. The probe is from 1 mm diameter to 15 mm diameter. Typically the device provide an ultrasonic signal of 800kHz to 3MHz with an output intensity Isata (spatial average temporal average intensity) ranging from 0,5 W/cm² to 5 W/cm². The signal is delivered for a period ranging from 20 sec to 5 min. The ultrasound device may be delivered in a pulse-echo mode or in a continuous mode.

The combination of the ultrasound device with a real-time ultrasound biomicroscopy (UBM) imaging system may be particularly suitable to allow visualization of the ciliary region and to guide needle injection more precisely for local gene delivery.

The microbubbles are destroyed in the sonification zone, releasing their contents. Cavitation also creates small shock waves that increase cell permeability enabling delivery of the therapeutic nucleic acid of interest. The use of microbubbles may also limit the amount of inflammatory response and may allow repeated injections. The gas-filled microspheres effectively lower the energy threshold for non-thermal cavitation.

Examples of ocular diseases that may be treated by the method of the present invention include ocular proliferative diseases, ocular neurodegenerative diseases, glaucoma, ocular infectious diseases, ocular inflammatory diseases (such as conjunctivitis, keratitis, endothelitis, uveitis, choroiditis, retinitis, retinochoroiditis, anterior uveitis, and inflammatory optic neuropathies), retinal degenerations (in particular retinitis pigmentosa, peripheral retinal degeneration, macular degeneration such as dry age-related macular degeneration), ischemic retinopathy (in particular retinopathy of prematurity and diabetic retinopathy), retinal vascular diseases, ocular ischemia syndrome and other vascular anomalies, choroidal disorders and tumors, vitreous disorders, glial proliferation such as proliferative vitreo retinopathy and glial proliferation associated to diabetic pre retinal engiogenesis, etc. Major diseases that may be prevented or treated by the present invention are described below.

Intraocular inflammation regroup all types of inflammation of the intraocular tissues, mainly uvea and retina. Intraocular inflammations may be from immunologic causes, infectious causes, iatrogenic causes or of unknown etiologies. They may be acute, recurrent or chronic. Intraocular inflammations are among the most causes of curable blindness. Posterior segment intraocular inflammations may be associated to vasculitis, optic neuritis, vitritis and chorea retinitis.

Inherited retinal dystrophies or retinitis pigmentosa are inherited blinding diseases due to mutations or deletions in gene implicated in the visual cycle. They begin in the young age and progress slowly until total blindness. Loss of photoreceptors is associated to loss of retinal pigment cells and to vascular and optic nerve atrophy at the later stages. Some of these inherited degeneration are due to mutation in mitochondrial DNA.

There are two major types of glaucoma: chronic glaucoma or primary open-angle glaucoma (POAG) and acute closed-angle glaucoma. Other variations include congenital glaucoma, pigmentary glaucoma, neovascular glaucoma and secondary glaucoma. Glaucoma is similar to ocular hypertension but with accompanying optic nerve damage and vision loss. Glaucoma is usually treated with eye drops, laser, or conventional eye surgery. If not treated, glaucoma will cause blindness.

Angiogenesis is the formation of new capillary blood vessels leading to neovascularization. Angiogenesis is a complex process which includes a series of sequential steps including endothelial cell mediated degradation of vascular basement membrane and interstitial matrices, migration of endothelial cells, proliferation of endothelial cells, and formation of capillary loops by endothelial cells. Though angiogenesis is a normal process for the development or maintenance of the vasculature, pathological conditions (i.e., angiogenesis dependent diseases) arise where blood vessel growth is actually harmful. Angiogenesis is notably associated with important diseases of ocular tissue, including diabetic retinopathies, age related macular degeneration, retinopathy of prematurity, corneal graft rejection, neovascular glaucoma and corneal scaring. Any abnormal growth of blood vessels in the eye can scatter and block the incident light prior to reaching the retina. Neovascularization can occur at almost any site in the eye and significantly alter ocular tissue function. Some of the most threatening ocular neovascular diseases are those which involve the retina. For example, many diabetic patients develop a retinopathy which is characterized by the formation of leaky, new blood vessels on the anterior surface of the retina and in the vitreous causing proliferative vitreoretinopathy. A subset of patients with age related macular degeneration develop subretinal neovascularization which leads to their eventual blindness.

Diabetic Retinopathy occurs when the retinal vessels inside the eye leak blood and fluids into the surrounding tissue. About 80% of patient with diabetes develop diabetic retinopathy. This disease is generally treated using a laser. However, laser therapy involves complications including retinal vein occlusion, loss of visual acuity, vitreous hemorrhage and sometimes fails. If left untreated, diabetic retinopathy may cause blindness.

Retinopathy of Prematurity (ROP) affects prematurely born babies. It consists of the abnormal growth of blood vessels within the retinal and vitreous. Progression to later stages of ROP can lead to the formation of scar tissue on the retina, vitreous hemorrhage, and retinal detachment. The treatment is usually performed either by laser or cryotherapy (freezing).

Ischemic retinopathies are retinopathies associated to vascular occlusion (capillaries or large vessels) that lead to neuroretinal suffering, cell death and neo angiogenesis. Macular degeneration is a disease that affects central vision and leads to loss of vision. Although there are forms of macular degeneration that strike young people, the condition occurs most commonly in people who are over 60 years of age. This disorder is thus called age-related macular degeneration (AMD). Because only the center of a person's vision is usually affected, blindness rarely occurs from the disease. However, injury to the macula in the center of the retina can destroy the ability to see straight ahead clearly. Dry forms associate degeneration of neuroretina, RPE cells and choroids. Wet forms associate previously described phenomenons and growth of neovessels from the choriocapillaries and/or retinal vessels, sub retinal detachment and hemorrhages, sub epithelial hemorrhages and tears, etc. Macular degeneration usually occurs after the age of sixty. While your central vision is reduced, most patients retain some vision and never go totally blind.

Keratitis is an inflammation of the cornea. Keratitis can be caused by bacterial, viral, or fungal infections, dry eyes resulting from disorders of the eyelid or diminished ability to form tears, exposure to very bright light, foreign objects that injure or become lodged in the eye, sensitivity or allergic reactions to eye makeup, dust, pollen, pollution, or other irritants and vitamin A deficiency.

Macular pucker (also called epiretinal membrane, retinal wrinkling, premacular fibrosis, and cellophane maculopathy) is due most often to age-related shrinkage of the vitreous which pulls away from the retina, causing the retina to scar and wrinkle. Other causes of macular pucker include trauma (from surgery or an eye injury), retinal detachment, inflammation, and problems with the retinal blood vessels. The only treatment is surgery which consists of a vitrectomy (removal of the vitreous) combined with peeling away of the scar tissue. The most common complication of vitrectomy is an increase in the rate of cataract development.

The treated eye disease may be chosen from scleritis, conjunctivitis, keratitis, endothelitis, uveitis, choroiditis, retinitis, retinochoroiditis, anterior uveitis, retinopathy of prematurity, diabetic retinopathy, proliferative vitreo retinopathy, inherited retinal dystrophies, age-related macular degeneration, open angle glaucoma, neovascular glaucoma, ischemic retinopathy, etc..

A particular aspect of the invention is a composition for use in a method of treating chronic uveitis comprising delivering to the ciliary muscle of a subject suffering therefrom a nucleic acid encoding a soluble receptor for TNF alpha.

Another particular aspect of the invention is a composition for use in a method of treating intraocular neovessels or macular oedema comprising delivering to the ciliary muscle of a subject suffering therefrom a nucleic acid encoding an anti VEGF, an anti VEGF receptor or an anti PLGF.

A further particular aspect of the invention is a composition for use in a method of treating or delaying retinitis pigmentosa comprising delivering to the ciliary muscle of a subject suffering therefrom a nucleic acid encoding a neurotrophic factor as described above.

Another particular aspect of the invention is a composition for use in a method of treating diabetic retinopathy comprising delivering to the ciliary muscle of a subject suffering therefrom a nucleic acid encoding an anti IRS-1 or IGF-1.

The present invention also relates to a pharmaceutical composition formulated with echo-contrast agent microbubbles and a therapeutic nucleic acid of interest for use in a method for treating an ocular disease in a subject wherein said method comprises the steps consisting of i) delivering said pharmaceutical composition into the ciliary muscle of the subject and ii) exposing the region where the pharmaceutical composition was delivered to ultrasound to induce transfection of said therapeutic nucleic acid of interest into said ciliary muscle.

In accordance with compositions for use according to the present invention, kits for preventing or treating an ocular disease are envisioned. Devices and pharmaceutical composition according to the present invention may be supplied together in a kit. Within the kit, the components may be separately packaged or contained. Other components such as excipients, carriers, other drugs or adjuvants, instructions for administration of the active substance or composition, and administration or injection devices can be supplied in the kit as well. Instructions can be in a written, video, or audio form, can be contained on paper, an electronic medium, or even as a reference to another source, such as a website or reference manual.

The invention will be further illustrated by the following figures and examples.

### EXAMPLE:

### Material & Methods

**Animals:** Female Lewis rats, weighing 150 to 200 g, were purchased from Janvier (Le Genest-Saint-Isle, France) at 7 weeks of age and roomed in our facility for 1 week before inclusion in the study. Experiments were conducted in accordance with the Association for Research in Vision and Ophthalmology (ARVO) Statement for the Use of Animals in Ophthalmic and Vision Research. For experiments, rats were anesthetized by intramuscular injection of a mixture of Ketamine 1000 (80 mg/kg; Virbac, Carros, France) and Largactil (0.5 mg/kg; Sanofi-Aventis, Paris, France). At the end of the experiments, rats were sacrificed by carbon dioxide inhalation.

**Plasmids:** Two commercially available plasmids were used to dose and locate reporter gene expression in the ciliary muscle: the pCMV-Gluc-1 (Nanolight Technology, Pinetop, AZ, USA) encoding a secreted Gaussia luciferase under control of a cytomegalovirus (CMV) promoter, purchased from Clontech (Palo Alto, CA, USA), and the pVAX1-LacZ (Invitrogen, Carlsbad, CA, USA) containing the *LacZ* gene under control of a CMV promoter (Clontech, Palo Alto, CA, USA).

After amplification in *Escherichia coli DH5-alpha,* plasmids were purified with Nucleobond AX1000 kits (Macherey-Nagel GmbH & Co, KG) according to the manufacturer's instructions, and then prepared at appropriate concentrations, in order to inject 15µg of plasmids in 10µL of saline, *i.e.* 1,5µg/µL without MB or 3µg/µL with 50% MB

**Microbubbles (MB):** We used for all our experiments commercially available, ready-to-use, a second-generation microbubbles provided by Artison Corp., (Inola, OK, USA). The Artison MB is a lipid-shell US contrast agent filled with perfluorocarbon gas and measuring around 2.4 µm in diameter. The MB solution is composed of approximately 13x10⁸ microspheres per millimetre.

MB were prepared according to the manufacturer's instructions. Artison MB were first re-dispersed by gently shaking the vial for 10 seconds, until obtaining a homogenous milky white suspension after which, the desired volume of the dispersion was withdrawn from the vial. Then, the bubbles were added to the plasmid solution and mixed gently for 10 seconds to allow their combination immediately prior to their injection.

**Ultrasound apparatus:** Ultrasound (US) treatment was performed using a sonoporation system designed for research: the Sonitron 2000, from Artison (Inola, OK, USA) with a plane unfocused transducer operating at either 1 MHz or 3 MHz (switchable). A 3-mm diameter probe was selected to deliver the acoustic energy to a small area for rat experiments. The transducer had an output intensity *I*sata (spatial-average temporal-average intensity) ranging from 0 to 5 W/cm², adjustable in 0.1 W/cm² increments with duty cycles (percentage of on-time over one pulse period) varying from 5 to 100% and variable pulse-repetition-frequency. The advantage of a pulsed mode of US application is that adverse thermal effects can be reduced. The treatment time was adjustable from 0 to 20 minutes.

***In vivo* sonoporation to rat ciliary muscle:** For therapy experiments, the eye was held in position using a surgical sheet. Fifteen µg of plasmid DNA alone or mixed with MB was then injected under surgical microscopy in the ciliary muscle of both rat eyes using a 30-gauge disposable needle on a 100-µl microsyringe (BD-microfine syringe, NM Médical, Asnières, France). To reach the ciliary muscle just below the sclera posterior to the limbus (junction where the transparent cornea joins the white opaque sclera), the injection was performed in the temporal superior side of the limbus. Immediately after the injection, the ultrasound tip was applied directly over the ocular surface after interposition of a transparent coupling gel (Gelaser, Alcon, Rueil-Malmaison, France) and the region that has been injected was exposed to US. The US probe was not moved during treatment. Both eyes were treated using the same procedure by the same operator. In all experiments, the following US settings were applied: 1 MHz, 2W/cm² *I*sata for 2 minutes, duty cycle of 50% with a pulse width of 5 ms (on-time) and a pulse interval of 5 ms (off-time) and pulse-repetition-frequency of 100 Hz.

**Experimental design:** Two sets of experiments were carried out on a total of 36 Lewis rats. Both eyes from the same animal were treated in an identical manner.

The first set of experiments aimed at assessing the kinetics of luciferase reporter gene expression in the ciliary muscle at day 7 (n = 20 rats) and 30 (n = 12 rats) after sonoporation. For this purpose, 15 µg of pCMV-Gluc-1 were injected in the ciliary muscle of both eyes of all animals. After plasmid injection, four experimental groups were formed for the day 7 assay. In the first group, no additional treatment was performed to serve as controls (control group, n=10 eyes). In the second group, the plasmid injection was immediately followed by US exposure (US group, n=10 eyes). In the two last groups, plasmid DNA mixed with microbubbles were co-injected into the ciliary muscle followed by US treatment (US+MB group, n=12 eyes) or without US exposure (MB group, n=8 eyes). Similar groups: control group (n=8 eyes), US group (n=8 eyes) and US+MB group (n=8 eyes) were set up for the day 30 assay. The effect of microbubbles alone was not evaluated at this time point. One additional untreated rat was used as negative control for luciferase expression at each time point. All animals were sacrificed on day 7 or day 30 after treatment. Eyes were enucleated, then, ocular fluids (aqueous and vitreous humours) were removed for evaluation of luciferase activity by luminometry.

In the second sets of experiment, rat eyes (n = 4) were used to analyse the localization of the β-galactosidase activity, after sonoporation of the plasmid containing the *LacZ* gene into the ciliary muscle. Fifteen µg of pVAX1-LacZ were injected in the ciliary muscle of six eyes. Then, eyes were exposed either to ultrasound plus microbubbles (n=2 eyes), either to US (n=2 eyes) or to microbubbles alone (n=2 eyes). The remaining rat, receiving no injection or exposure to US, served as a control. At day 7 after transfection, eyes were enucleated and were prepared for histochemical analysis.

Eyes were examined by naked eye observation immediately after each experiment, and then 7 and 30 days after gene transfection.

**Measurement of luciferase activity:** At day 7 or 30 after treatment, the eyes treated with intramuscular injection of 15 µg pCMV-Gluc-1, with or without US and/or MB, were enucleated. Ocular fluids (aqueous and vitreous humours) were removed and, after centrifugation at 5000g for 5 min at 4°C, supernatants were kept at -20°C until used.

The luciferase activity was assessed on 10 µl from each sample placed in a white-96-well plate (Costar®, tissue-culture treated, white plate) with *Renilla* Luciferase Assay System according to manufacturer's protocol (Promega, Charbonnières, France). The detector was a luminometer Wallac Victor2, 1420 Multi-label Counter (EG&G Wallac, Evry, France) which adds 50 µl of luciferase assay substrate (Promega) to the sample and integrates the light produced by the sample over 10 seconds with 2-second delay. Data analysis was performed using the Wallac 1420 workstation software. Results are given for each sample in counts per second (cps). Background luminescence was less than 120 cps.

**Localization of beta-galactosidase expression in the ciliary *region: in situ* histochemical analysis:** Seven days after treatment, the eyes were enucleated, fixed in 2% paraformaldehyde and 0.2% glutaraldehyde in phosphate-buffered saline (PBS) for 1 hour at 4 °C, then rinsed three times in PBS. The whole eyes were subsequently incubated overnight in the dark at room temperature (37°C) in the presence of a chromogenic X-gal reagent (1mg/ml of 5-bromo-4-chloro-3-indolyl-d-galactopyranoside, Sigma, Saint-Quentin-Fallavier, France) in PBS containing 5 mM of K3Fe(CN)6, 5mM of K4Fe(CN)6, 2mM of MgCl2 and 0.02% NP-40for colorimetric detection of β-galactosidase activity. Next, the eyes were embedded in paraffin, and then sliced using a Microm HM-340E microtome (Leica Microsystems, Nussloch GmbH, Germany). Longitudinal 10-µm thick sections were cut parallel to the optic axis. One half of paraffin sections from each sample was mounted in glycerol/PBS (1:1) and the other half was counterstained with haemalum-eosin to stain the nuclei and cytoplasms, respectively. During examination under a Leitz Aristoplan photomicroscope, images were captured using a Leica DFC 480 digital camera with either a 10x or 25x objective lens and an exposure time kept respectively at 2.55msec and 14.9msec.

**Statistical analysis:** The program used for the analysis of the data was GraphPad Prism (GraphPad Software, San Diego, CA, USA). Results are expressed as mean ± standard error of the mean (SEM). Data analysis was performed using a non-parametric one-way analysis of variance (ANOVA, Kruskal-Wallis test) followed by a Dunn's multiple comparison test. Prior to the ANOVA, extreme outlier values were identified by Grubb's test and excluded from the dataset (one point per group). Statistical significance was set *at p <* 0.05.

### Results:

Clinical examination of treated eyes immediately and on days 7 and 30 after treatment showed the absence of inflammation or gross ocular damage.

**Kinetics of luciferase activity in ocular fluids:** *Gaussia-luciferase* luminescence was shown in the rat ocular fluids, 7 days after injection of the pCMV-Gluc-1 plasmid (15 µg) into the ciliary muscle in four different treatment groups. The mean level of luciferase activity in ocular fluids was increased in both groups treated with US exposure, with or without MB, compared to the control group (DNA injection alone). In eyes that received US application after co-administration of plasmid and MB (n = 11), luciferase activity in ciliary muscle was significantly higher by approximately 2.6-fold compared to the control group (n = 9, p < 0.05) or to the MB group (n = 7, p < 0.005). In contrast, US alone (n = 9) induced a lower increase in luminescence level (of 1.5-fold compared to the control group with a non statistically significant difference). Only the difference with the MB group was statistically significant (p < 0.005). Little luciferase activity was detected without US exposure after injection of plasmid alone or mixed with MB. Our results show a high dispersion in luciferase activity, in particular for the US-treated groups.

On day 30, luciferase gene expression has dropped in each experimental group by around 50% between days 7 and 30 after treatment. ANOVA statistical analysis showed that the factor "time" has a very significant effect and accounts for 14.16% of the total variance (p=0.0029) and that the factor "group" has a significant effect and accounts for 10.84% of the total variance (p=0.0304). The interaction between both factors is considered not significant. Bonferroni post tests determined that luminescence was significantly decreased between D7 and D30 only in the "US+MB group" (p<0.01).

**Localization of β-Galactosidase expression in the ciliary region:** Seven days after ciliary muscle targeted sonoporation of a plasmid containing the *LacZ* reporter gene, the histochemical staining revealed that β-galactosidase expression (blue cells) was detected within the field of US application in the ciliary region cells. This method led to effective expression in the ciliary region in the case of US+MB application and US application, whereas no detectable β-galactosidase activity was observed in the ciliary region without any US application (control eye).

### Discussion:

The main objective of the present study was to investigate the feasibility of gene transfer into ciliary muscles by sonoporation. To our knowledge, our work is the first study on *in vivo* plasmid DNA transfer targeted to the ciliary muscle mediated by US and MB. In the eye, the ciliary smooth muscle offers two main advantages. First, it is an easily accessible tissue without penetration in the vitreous cavity. Second, this muscle is located between the anterior and posterior parts of the ocular sphere, allowing protein secretion towards the aqueous humor as well as the vitreous and retina. Our present experiments involving the co-administration of microbubbles and plasmid DNA were carried out with reporter genes widely used as markers to assess the efficiency of gene-therapy technology

We have shown that external ultrasound exposure on the ciliary region following a single intramuscular injection of a plasmid mixture containing a reporter gene and MB is a simple and effective method to facilitate *in vivo* gene transfer into the rat ciliary muscle. The observation of protein secretion in ocular fluids (aqueous vitreous and humours) at day 7 after sonoporation of 15 µg plasmid reporter delivered into the ciliary muscle confirms the feasibility of this method. Luciferase uptake into the muscle tissue was about 2.6-fold higher in US + MB group compared with controls treated with luciferase injection alone. When no US was delivered after DNA injection, gene expression was low.

In addition to the benefits of the combined use of ultrasound and microbubbles, our approach offers some advantages specific to ocular gene transfer by sonoporation. First, it requires only intramuscular needle for DNA injection and external US exposure, which is widely accepted in clinical practice. Its safety has been well-established. US can be focused with precision within the targeted site, even of small size as are ciliary muscles, to produce local gene delivery and are widely used for clinical examinations and therapies.

In summary, this study demonstrates that the ocular ciliary muscle can be targeted by DNA sonoporation allowing for protein secretion into the ocular sphere. Sonoporation targeted to ciliary muscles has potential as a non-viral, minimally-invasive, safe and efficient gene delivery procedure for the treatment of various ocular diseases.

### REFERENCES:

Throughout this application, various references describe the state of the art to which this invention pertains.

## Claims

1. A pharmaceutical composition formulated with echo-contrast agent microbubbles and a therapeutic nucleic acid of interest for use in a method for treating an ocular disease in a subject wherein said method comprises the steps consisting of i) delivering said pharmaceutical composition into the ciliary muscle of the subject and ii) exposing the region where the pharmaceutical composition was delivered to ultrasound to induce transfection of said therapeutic nucleic acid of interest into said ciliary muscle.

2. The pharmaceutical composition for use according to claim 1 wherein said therapeutic nucleic acid of interest is a desoxyribonucleic acid (DNA) molecule or a ribonucleic acid (RNA) molecule.

3. The pharmaceutical composition for use according to claim 1 or 2 wherein said therapeutic nucleic acid of interest is inserted in a vector, such as a plasmid.

4. The pharmaceutical composition for use according to any of claims 1 to 3 wherein said nucleic of interest encoded for a polypeptide selected from the group consisting of enzymes, blood derivatives, hormones, lymphokines, cytokines, chimiokines, antiinflammatory factors, growth factors, trophic factors, neurotrophic factors, haematopoietic factors, angiogenic factors, anti-angiogenic factors, inhibitors of metalloproteinase, regulators of apoptosis, coagulation factors, receptors thereof, in particular soluble receptors, a peptide which is an agonist or antagonist of a receptor or of an adhesion protein, antigens, antibodies, fragments or derivatives thereof and other essential constituents of the cell.

5. The pharmaceutical composition for use according to any of claims 1 to 4 wherein the microbubble shell comprises a polymer or a surfactant selected from the group consisting of lipids, including phospholipids and fluorinated lipids.

6. The pharmaceutical composition for use according to any of claims 1 to 5 wherein the gas incorporated in the microbubbles is a fluorocarbon compound, preferably a perfluorocarbon selected from the group consiting of perfluoroalkanes such as perfluoromethane, perfluoroethane, perfluoropropanes, perfluorobutanes, perfluoropentanes, perfluorohexanes or perfluoroheptanes; perfluoroalkenes such as perfluoropropene, perfluorobutenes, perfluorobutadiene, perfluoropentenes or perfluoro-4-methylpent-2-ene; perfluoroalkynes such as perfluorobut-2-yne; and perfluorocycloalkanes such as perfluorocyclobutane, perfluoromethylcyclobutane, perfluorodimethylcyclobutanes, perfluorotrimethylcyclobutanes, perfluorocyclopentane ,perfluoromethylcyclopentane, perfluorodimethylcyclopentanes, perfluorocyclohexane, perfluoromethylcyclohexane or perfluorocycloheptane.

7. The pharmaceutical composition for use according to any of claims 1 to 6 wherein ultrasound is delivered by an ultrasound device providing an ultrasonic signal of 800kHz to 3MHz with an output intensity Isata ranging from 0,5 W/cm² to 5 W/cm².

8. The pharmaceutical composition for use according to any of claims 1 to 7 wherein said ocular disease is selected from the group consisting of ocular proliferative diseases, ocular neurodegenerative diseases, glaucoma, ocular infectious diseases, ocular inflammatory diseases such as conjunctivitis, keratitis, endothelitis, uveitis, choroiditis, retinitis, retinochoroiditis, anterior uveitis, and inflammatory optic neuropathies, retinal degenerations, in particular retinitis pigmentosa, peripheral retinal degeneration, macular degeneration such as dry age-related macular degeneration, ischemic retinopathy, in particular retinopathy of prematurity and diabetic retinopathy, retinal vascular diseases, ocular ischemia syndrome and other vascular anomalies, choroidal disorders and tumors, vitreous disorders, glial proliferation such as proliferative vitreo retinopathy and glial proliferation associated to diabetic pre retinal angiogenesis.

## Patentansprüche

1. Pharmazeutische Zusammensetzung, die mit Echokontrast-mittel-Mikrobläschen und einer therapeutischen Nukleinsäure von Interesse zur Verwendung in einem Verfahren zur Behanglung einer Augenerkrankung bei einem Patienten formuliert ist, wobei das Verfahren die Schritte umfasst, die daraus bestehen, dass i.) die pharmazeutische Zusammensetzung in den Ziliarmuskel des Patienten befördert wird und ii.) der Bereich, in den die pharmazeutische Zusammensetzung befördert wurde, einem Ultraschall ausgesetzt wird, um eine Transfektion der therapeutischen Nukleinsäure von Interesse in den Ziliarmuskel zu induzieren.

2. Pharmazeutische Zusammensetzung zur Verwendung nach Anspruch 1, wobei die therapeutische Nukleinsäure von Interesse ein Desoxyribonukleinsäure-(DNA)-Molekül oder ein Ribonukleinsäure-(RNA)-Molekül ist.

3. Pharmazeutische Zusammensetzung zur Verwendung nach Anspruch 1 oder 2, wobei die therapeutische Nukleinsäure von Interesse in einen Vektor, wie ein Plasmid, eingefügt ist.

4. Pharmazeutische Zusammensetzung zur Verwendung nach einem der Ansprüche 1 bis 3, wobei die Nuklein von Interesse für ein Polypeptid kodiert ist, das aus der Gruppe ausgewählt ist, bestehend aus: Enzymen, Blutderivaten, Hormonen, Lymphokine, Zytokine, Chemokine, entzündungshemmende Faktoren, Wachstumsfaktoren, Nahrungsfaktoren, neurotrophen Faktoren, blutbildenden Faktoren, angiogenen Faktoren, anti-angiogenen Faktoren, Inhibitoren von Metalloproteinasen, Regulatoren von Apoptose, Gerinnungsfaktoren, Rezeptoren davon, insbesondere lösbare Rezeptoren, ein Peptid, das ein Agonist oder ein Antagonist eines Rezeptors oder eines Adhäsionsproteins ist, Antigene, Antikörper, Fragmente oder Derivative davon und andere wesentliche Bestandteile der Zelle.

5. Pharmazeutische Zusammensetzung zur Verwendung nach einem der Ansprüche 1 bis 4, wobei die Mikrobläschenhülle ein Polymer oder ein Tensid umfasst, die aus der Gruppe ausgewählt sind, bestehend aus: Lipiden, einschließlich Phospholipiden und fluorierten Lipiden.

6. Pharmazeutische Zusammensetzung zur Verwendung nach einem der Ansprüche 1 bis 5, wobei das in den Mikrobläschen enthaltene Gas eine Fluorkohlenstoffverbindung ist, vorzugsweise ein Perfluorkohlenstoff, der der Gruppe ausgewählt ist, bestehend aus: Perfluoralkanen, wie Perfluormethan, Perfluorethan, Perfluorpropan, Perfluorbutan, Perfluorpentan, Perfluorhexan, oder Perfluorheptan; Perfluoralkenen, wie Perfluorpropen, Perfluorbuten, Perfluorbutadien, Perfluorpenten oder Perfluor-4-Methylpent-2-ene; Perfluoralkinen, wie Perfluorbut-2-ine; und Perfluorcycloalkanen, wie Perfluorcyclobutan, Perfluormethylcyclobutan, Perfluordimethylcyclobutane, Perfluortrimethylcyclobutane, Perfluorcyclopentan, Perfluormethylcyclopentan, Perfluordimethylcyclopentane, Perfluorcyclohexan, Perfluormethylcyclohexan oder Perfluorcycloheptan.

7. Pharmazeutische Zusammensetzung zur Verwendung nach einem der Ansprüche 1 bis 6, wobei ein Ultraschall von einer Ultraschallvorrichtung geliefert wird, die ein Ultraschallsignal von 800 kHz bis 3 MHz mit einer Ausgangsintensität Isata, die im Bereich von 0,5 W/cm² bis 5 W/cm² liegt, bereitstellt.

8. Pharmazeutische Zusammensetzung zur Verwendung nach einem der Ansprüche 1 bis 7, wobei die Augenerkrankung aus der Gruppe ausgewählt ist, bestehend aus: proliferative Augenerkrankungen, neurodegenerative Augenerkrankungen, grüner Star, infektiöse Augenerkrankungen, entzündliche Augenerkrankungen, wie Bindehautentzündung, Hornhautentzündung, Endotheliitis, Uveitis, Chorioiditis, Netzhautentzündung, Retinochorioiditis, anteriore Uveitis, entzündliche Optikus-neuropathie, Netzhautdegeneration, insbesondere Retinis pigmentosa, periphere Netzhautdegeneration, Makuladegeneration wie eine trockene altersbedingte Makuladegeneration, ischemische Retinopathie, insbesondere Frühgeborenen-Retinopathie und diabetische Retinopathie, retinale Gefäßerkrankungen, Augenischemiesyndrom und andere Gefäßanomalien, choroidal Störungen und Tumore, Glaskorperstörungen, Glia-Proliferation wie eine proliferative Vitreo-Retinopathie und eine mit einer diabetischen präretinalen Angiogenese verknüpften Glia-Pro-liferation.

## Revendications

1. Composition pharmaceutique formulés des microbulles d'un agent de contraste échographique et un acide nucléique thérapeutique d'intérêt, pour son utilisation dans un procédé de traitement d'une maladie oculaire chez un sujet, où ledit procédé comprend les étapes consistant à i) administrer ladite composition pharmaceutique dans le muscle ciliaire du sujet et ii) exposer la région dans laquelle la composition pharmaceutique a été administrée à des ultrasons afin d'induire une transfection, dudit acide nucléique thérapeutique d'intérêt dans ledit muscle ciliaire.

2. Composition pharmaceutique pour son utilisation selon la revendication 1, où ledit acide nucléique thérapeutique d'intérêt est une molécule d'acide désoxyribonucléique (ADN) ou une molécule d'acide ribonucléique (ARN),

3. Composition pharmaceutique pour son utilisation selon la revendication 1 ou 2, où ledit acide nucléique thérapeutique d'intérêt est inséré dans un vecteur, tel qu'un plasmide.

4. Composition pharmaceutique pour son utilisation selon l'une quelconque des revendications 1 à 3, où ledit nucléique d'intérêt code pour un polypeptide sélectionné dans le groupe consistant en des enzymes, des dérivés du sang, des hormones, des lymphokines, des cytokines, des chimiokines, des facteurs anti-inflammatoires, des facteurs de croissance, des facteurs trophiques, des facteurs neurotrophiques, des facteurs hématopoïétiques, des facteurs angiogéniques, des facteurs anti-angiogéniques, des inhibiteurs de métalloprotéinases, des régulateurs de l'apoptose, des facteurs de la coagulation, des récepteurs de ceux-ci, en particulier des récepteurs solubles, un peptide qui est un agoniste ou un antagoniste d'un récepteur ou d'une protéine d'adhésion, des antigènes, des anticorps, des fragments ou des dérivés de ceux-ci et d'autres constituants essentiels de la cellule.

5. Composition pharmaceutique pour son utilisation selon l'une quelconque des revendications 1 à 4, où l'enveloppe des microbulles comprend un polymère ou un tensioactif sélectionné dans le groupe consistant en des lipides, comprenant des phospholipides ou des lipides fluorées.

6. Composition pharmaceutique pour son utilisation selon l'une quelconque des revendications 1 à 5, où le gaz incorporé dans les microbulles est un composé fluorocarboné, de préférence un perfluorocarbone sélectionné dans le groupe consistant en des perfluoroalcanes tel que le perfluoromêthane, le perfluoroéthane, des perfluoropropanes, des perfluorobutanes, des perfluoropentanes, des perfluorohexanes ou des perfluoroheptanes ; des perfluoroalcènes tel que le perfluoropropène, des perfluorobutènes, le perfluorobutadiène, des perfluoropentènes ou le perfluoro-4-méthylpent-2-ène ; des perfluoroalcynes tel que le perfluorobut-2-yne ; et des perfluorocycloalcanes tel que le perfluorocyclobutane, le perfluorométhylcyclobutane, des perfluorodiméthylcyclobutanes, des perfluorotriméthylcyclobutanes, le perfluorocyclopentane, le perfluorométhylcyclopentane, des perfluorodiméthylcyclopentanes, le perfluorocyclohexane, le perfluoromêthylcyclohexane ou le perfluorocycloheptane.

7. Composition pharmaceutique pour son utilisation selon l'une quelconque des revendications 1 à 6, où l'ultrason est délivré par un dispositif ultrasonore fournissant un signal ultrasonore de 800 kHz à 3 MHz avec une intensité de sortie Isata comprise entre 0,5 w/cm² et 5 w/cm²˙

8. Composition pharmaceutique pour son utilisation selon l'une quelconque des revendications 1 à 7, où ladite maladie oculaire est sélectionnée dans le groupe consistant en des maladies oculaires prolifératives, des maladies oculaires neurodégénératives, le glaucome, des maladies oculaires infectieuses, des maladies oculaires inflammatoires comme la conjonctivite, la kératite, l'endothélite, l'uvéite, la choroïdite, la rétinite, la rétinochoroïdite, l'uvéite antérieure, et des neuropathies optiques inflammatoires, des dégénérescences rétiniennes, en particulier la rétinite pigmentaire, la dégénérescence rétinienne périphérique, la dégénérescence maculaire tel que la dégénérescence maculaire liée à l'âge de type sec, la rétinopathie ischémique, en particulier la rétinopathie du prématuré et la rétinopathie diabétique, des maladies vasculaires rétiniennes, le syndrome d'ischémie oculaire et d'autres anomalies vasculaires, des désordres et des tumeurs choroïdiennes, des désordres vitréennes, une prolifération gliale comme la vitréorétinopathie proliférante et une prolifération gliale associée à une angiogenèse pré-rétinienne diabétique.
